# EUROPEAN PATENT APPLICATION

(11) **EP 4 375 372 A1**
(43) Date of publication of application: **29.05.2024**
(21) Application number: 22836737.1
(22) Date of filing: 24.06.2022
(51) Int. Cl.: C12N 9/88, C12N 15/60, C12N 15/70, C12N 1/21, A61K 38/51, A61P 31/04, C12R 1/19

(54) **ANTIBACTERIAL PEPTIDE P104 AND LYASE LYSP53 HAVING BROAD-SPECTRUM CRACKING ACTIVITY, AND APPLICATIONS THEREOF**

(30) Priority: 05.07.2021 CN 202110760643
(71) Applicant: Wuhan Institute Of Virology, Chinese Academy of Sciences, Wuhan, Hubei 430071 (CN)
(72) Inventor: WEI, Hongping, Wuhan, Hubei 430071 (CN); YANG, Hang, Wuhan, Hubei 430071 (CN); LI, Changchang, Wuhan, Hubei 430071 (CN); YU, Junping, Wuhan, Hubei 430071 (CN); JIANG, Mengwei, Wuhan, Hubei 430071 (CN)
(74) Representative: Hellmich, Wolfgang
(86) International application number: PCT/CN2022/100968
(87) International publication number: WO 2023/279983

(57) **Abstract**

An antibacterial peptide P104 and a lysin LysP53 with broad-spectrum lytic activity and applications thereof are provided. The amino acid sequence of the antibacterial peptide P104 is shown as SEQ ID NO: 1, and the gene sequence of the antibacterial peptide P104 is shown as SEQ ID NO: 3. The amino acid sequence of the lysin LysP53 is shown as SEQ ID NO: 2, and the gene sequence of the lysin LysP53 is shown as SEQ ID NO: 4. The antibacterial peptide P104 and the lysin LysP53 have great lytic effects on *Acinetobacter baumannii, Pseudomonas aeruginosa, Klebsiella pneumoniae* and *Escherichia coli in vitro,* and can lyse Gram-negative bacteria in a broad spectrum. The lysin LysP53 can be subjected to soluble expression in *Escherichia coli,* and the activity of the lysin is high. Therefore, they have good application prospects in the research and development of anti-infective drugs.

## Description

### TECHNICAL FIELD

The disclosure relates to the field of biological agents, and more particularly to an antibacterial peptide P104 and a lysin LysP53 with broad-spectrum lytic activity, and applications thereof.

### BACKGROUND

Antibiotics were once considered as the most powerful weapon to treat bacterial infectious diseases. However, with the increasing abuse of antibiotics and the increasing problem of bacterial resistance, the speed of research and development of new antibiotics is much lower than that of drug-resistant bacteria. There are few new antibiotics that can be used as substitutes, and the cost is expensive, which causes a large number of clinical drug-resistant bacteria-infected people to die because there is no medicine to cure. Under such a severe situation of antibiotic resistance, bacteriophages that can be used as substitutes for antibiotics to become a new type of antibacterial preparation have attracted the attention of scholars at home and abroad.

Bacteriophages are a class of viruses that use microorganisms (bacteria, fungi, actinomycetes, or spirochetes) as hosts, have no cell structure, consist only of capsid proteins and their internal genetic material, and must rely on the host for replication and proliferation. Bacteriophages, as natural killers of bacteria, are widely found in nature and are the most abundant and diverse organisms on earth, and their number is about 10 times that of bacteria. For more than a century, the safety and effectiveness of bacteriophage therapy have been confirmed in a large number of animal experiments. Lytic bacteriophages immediately initiate the expression of their own early genes after infection of host bacterial cells, and degrade the host deoxyribonucleic acid (DNA) through these early gene expression products, thus terminating the host gene expression and seizing the host DNA replication machine to synthesize their own nucleic acids in large quantities. When enough bacteriophages are produced, these nucleic acids are used as templates to produce a large number of bacteriophage structural proteins, which process and package the bacteriophage genome to produce the next generation of bacteriophage particles. Once the bacteriophage regulatory protein accumulates to a certain level, it will start the expression of bacteriophage perforin in large quantities, and the perforin will form holes in the host cell, which will cause bacteriophage lysin to pass through the inner membrane and approach the cell wall to lyse the cell wall, thereby achieving the purpose of lysing the bacteria.

In addition to intact bacteriophage particles as antibacterial agents, peptidoglycan hydrolase encoded by the bacteriophage, lysin, is also a new type of antibacterial molecule with great development potential. Lysin is a kind of hydrolase encoded by double-stranded DNA bacteriophage in the late stage of infection with the host, which can hydrolyze peptidoglycan of the bacterial cell wall, resulting in bacterial lysis and release of offspring bacteriophage. Gram-positive bacterial lysins have been systematically and maturely studied. Many natural lysins and chimeric lysins have been proven to be safe and effective in animal infection models, among which several lysins against drug-resistant *Staphylococcus aureus* (*S*. *aureus)* infection have entered the clinical trial stage. The advantages of lysin, such as high efficiency, specificity, low drug resistance and synergistic effect with existing antibiotics, make it a new and promising antibacterial drug.

At present, the application of bacteriophage lysin is mainly to lyse Gram-positive bacteria, such as *Staphylococcus aureus, Listeria monocytogenes* and *Enterococcus,* but there are few studies on bacteriophage lysin with lytic activity to Gram-negative bacteria. Therefore, it is necessary to develop a new bacteriophage lysin with a high lytic activity against Gram-negative bacteria.

### SUMMARY

Aiming to overcome the shortcomings of the related art, the disclosure provides an antibacterial peptide P104 and a lysin LysP53 with broad-spectrum lytic activity, and applications thereof. The antimicrobial peptide P104 and the lysin LysP53 have good lytic effects on *Acinetobacter baumannii, Pseudomonas aeruginosa, Klebsiella pneumoniae* and *Escherichia coli in vitro,* and can lyse Gram-negative bacteria in a broad spectrum.

An objective of the disclosure is to provide an antibacterial peptide P104 with broad-spectrum lytic activity.

Specifically, the antibacterial peptide P104 with broad-spectrum lytic activity is provided, where the amino acid sequence of the antibacterial peptide P104 is shown as SEQ ID NO: 1.

In an embodiment, the gene sequence of the antibacterial peptide P104 is shown as SEQ ID NO: 3.

Another objective of the disclosure is to provide a lysin LysP53 with broad-spectrum lytic activity.

Specifically, the lysin LysP53 with broad-spectrum lytic activity includes the antibacterial peptide P104, and the amino acid sequence of the lysin LysP53 is shown as SEQ ID NO: 2.

In an embodiment, the gene sequence of the lysin LysP53 is shown as SEQ ID NO: 4.

In an embodiment, primer sequences configured to amplify the nucleotide sequence of LysP53 are shown as SEQ ID NO: 5 and SEQ ID NO: 6.

The disclosure also provides a recombinant expression vector including the gene sequence of the lysin LysP53.

In an embodiment, the recombinant expression vector including the gene sequence of the lysin LysP53 is a prokaryotic expression vector pET28a-LysP53.

The disclosure also provides a host cell including the recombinant expression vector.

In an embodiment, the host cell including the recombinant expression vector is *Escherichia coli* BL21(DE3).

The disclosure also provides a preparation method of the lysin LysP53 includes the following steps:
S1, amplifying a lysin LysP53 gene from a bacteriophage p53 genome;
S2, constructing a recombinant expression vector pet28a-LysP53 expressing the lysin Lysp53 gene;
S3, transforming the recombinant expression vector pET28a-LysP53 into competent cells of *Escherichia coli* BL21(DE3) to obtain a transformed product, and screening the transformed product to obtain engineering bacteria expressing the lysin LysP53 gene;
S4, inducing expression of the engineering bacteria by using isopropyl β-D-thiogalactopyranoside (IPTG) to obtain a recombinant gene expression product; and
S5, purifying and separating the recombinant gene expression product by a nickel column to obtain the lysin LysP53.

The disclosure also provides an application of the antibacterial peptide P104 with broad-spectrum lytic activity and/or the lysin LysP53 with broad-spectrum lytic activity in lysing Gram-negative bacteria.

In an embodiment, the Gram-negative bacteria include *Acinetobacter baumannii, Pseudomonas aeruginosa, Klebsiella pneumoniae* and *Escherichia coli.*

Compared with the related art, the disclosure has the following advantages.

The disclosure provides the antibacterial peptide P104 with broad-spectrum lytic activity and the lysin LysP53 with broad-spectrum lytic activity, in which the antibacterial peptide P104 and the lysin LysP53 have good lytic effects on *Acinetobacter baumannii, Pseudomonas aeruginosa, Klebsiella pneumoniae* and *Escherichia coli in vitro,* and can broadly lyse Gram-negative bacteria. In addition, the lysin LysP53 can be subjected to soluble expression in *Escherichia coli,* and the activity of the lysin is high. Therefore, the antibacterial peptide P104 and the lysin LysP53 have good application prospects in the research and development of anti-infective drugs.

### BRIEF DESCRIPTION OF DRAWINGS

In order to illustrate technical solutions in embodiments of the disclosure more clearly, the drawings needed in the description of the embodiment will be briefly introduced below. Apparently, the drawings in the following description are merely some embodiments of the disclosure. For those skilled in the art, other drawings can be obtained according to these drawings without creative work.
FIG. 1 illustrates a chromatogram of an antibacterial peptide P104 showing its purity according to an embodiment 1 of the disclosure.
FIG. 2 illustrates a gel image of sodium dodecyl sulfate-polyacrylamide gel electrophoresis (SDS-PAGE) of the lysin LysP53 after purification according to an embodiment 2 of the disclosure.
FIG. 3 illustrates a diagram showing time-dependent results of the lysin LysP53 lysing *A*. *baumannii* WHG40137 according to an embodiment 3 of the disclosure.
FIG. 4 illustrates a diagram showing results of lytic activity of the lysin LysP53 lysing the *A. baumannii* WHG40137 at different growth stages according to an embodiment 4 of the disclosure.
FIG. 5 illustrates a diagram showing results of lytic activity of the lysin LysP53 at different pH values according to an embodiment 5 of the disclosure.
FIG. 6 illustrates a diagram respectively showing broad spectrum of the lysin LysP53 lysing different Gram-negative bacterial strains *in vitro* according to an embodiment 6 of the disclosure.
FIG. 7 illustrates a diagram showing results of lytic activity of the lysin LysP53 in killing the *A. baumannii* WHG40137 at different ethylenediaminetetraacetic acid (EDTA) concentrations according to an embodiment 7 of the disclosure.
FIG. 8 illustrates a diagram showing results of lytic activity of the lysin LysP53 in killing the *A. baumannii* WHG40137 on skin surfaces of mice according to an embodiment 8 of the disclosure.
FIG. 9 illustrates a diagram showing broad spectrum of the antibacterial peptide P104 and the lysin LysP53 *in vitro* according to the embodiment 8 of the disclosure.

### DETAILED DESCRIPTION OF EMBODIMENTS

Technical solutions in embodiments of the disclosure will be described clearly and completely with reference to the attached drawings. Apparently, the described embodiments are merely some of the embodiments of the disclosure, but not all the embodiments. Based on the embodiments in the disclosure, all other embodiments obtained by those skilled in the art without creative labor belong to the scope of protection of the disclosure.

Unless otherwise specified, reagents, Gram-negative bacteria strains and equipment used in the disclosure are commercially available.

Unless otherwise specified, the experimental methods used in the disclosure are all conventional experimental methods. The primers and sequencing work used are completed in Sangon Biotech (Shanghai) Co., Ltd. The antimicrobial peptide P104 is synthesized in ChinaPeptides (QYAOBIO) Biotechnology Company.

The gene sequence of the antimicrobial peptide P104 and the gene sequence of the lysin LysP53 of the disclosure are derived from the genome of bacteriophage P53, and the genome sequence has been uploaded to the website of the National Center for Biotechnology Information (NCBI, https://www.ncbi.nlm.nih.gov/) with the accession number of MW590698.

### Embodiment 1 Synthesis of antibacterial peptide P104

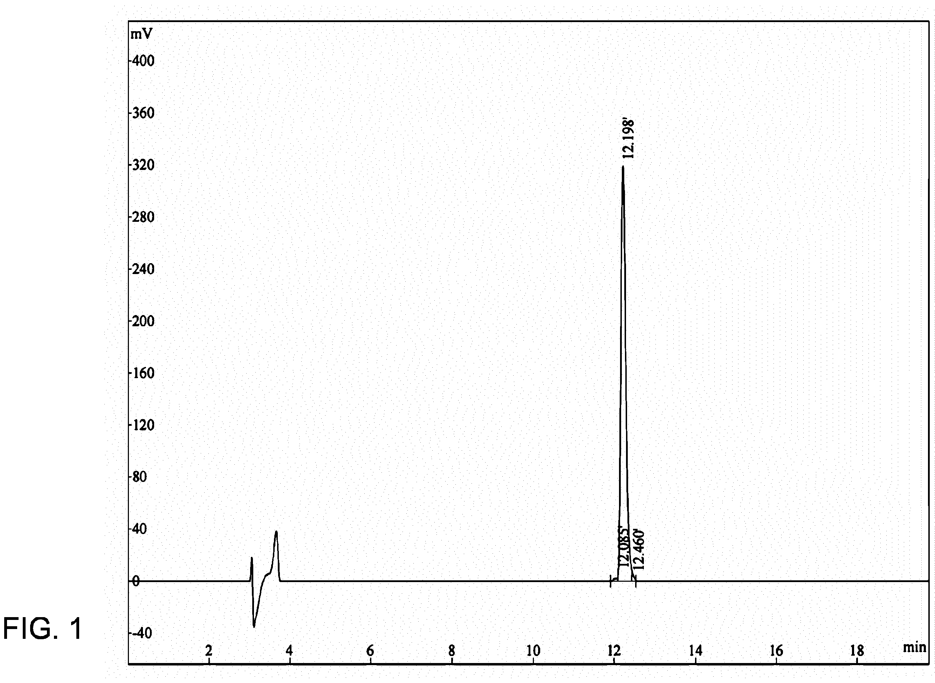

After a lot of experiments and analysis, the inventor found an antibacterial peptide P104 from the genome of bacteriophage P53, the amino acid sequence of the antibacterial peptide P104 is shown as SEQ ID NO: 1; and the gene sequence of the antibacterial peptide P104 is shown as SEQ ID NO: 3. The antibacterial peptide P104 is synthesized by ChinaPeptides (QYAOBIO) Biotechnology Company, the purity of the synthesized antibacterial peptide P104 is 98.58%, and the molecular weight is 3885.57. The purity of the antibacterial peptide P104 is determined by high-performance liquid chromatography, and the chromatographic purity diagram of the antibacterial peptide P104 is shown in FIG. 1, and the analysis results are shown in Table 1 below.

**Table 1 List of components of antimicrobial peptide P104 detected by liquid chromatography**

| Retention time (minute abbreviated as min) | Peak area | Purity (%) |
|---|---|---|
| 12.085 | 19368 | 0.7814 |
| 12.198 | 2443361 | 98.58 |
| 12.460 | 15928 | 0.6426 |
| Total | 2478657 | 100 |

The liquid phase conditions of high-performance liquid chromatography are as follows.

Chromatographic column: Kromasil^{®} 100-5-C18 (4.6 millimeters abbreviated as mm × 150 mm, 5 micrometers abbreviated as µm); mobile phase: a mobile phase A is an acetonitrile solution containing 0.1% (volume per volume abbreviated as v/v) trifluoroacetic acid (TFA) and a mobile phase B is an aqueous solution containing 0.1% (v/v) TFA, and gradient elution (GE) is carried out, and the gradient elution program is shown as Table 2. The flow rate is 1.0 milliliter per minute (mL/min), the column temperature is 25 Celsius degree (°C), the sample volume is 10 microliters (µL), and the detection wavelength is 220 nanometers (nm).

**Table 2 Gradient elution program table**

| Time (min) | Mobile phase A (%) | Mobile phase B (%) |
|---|---|---|
| 0 | 20 | 80 |
| 20 | 50 | 50 |
| 20.1 | 100 | 0 |

### Embodiment 2 Expression and purification of lysin LysP53

After a lot of experiments and analysis, the inventor found a fragment of the lysin LysP53 from the genome of bacteriophage p53, and the amino acid sequence of the lysin LysP53 is shown as SEQ ID NO: 2; and the gene sequence of the lysin LysP53 is shown as SEQ ID NO: 4.

### 2.1 Construction of recombinant expression vector

According to the gene sequence of the lysin LysP53, the following primer sequences are designed by using the conventional primer design software in the related art:
53P37-F: ctttaagaaggagatataccatggATGACGATGACAACAAAACGTA (with Ncol restriction site as shown as SEQ ID NO: 5); and
53P37-R: tggtggtggtggtggtgctcgagCCCCGCCAATTCAAAGTGTGGGCT (with *Xho*l restriction site as shown as SEQ ID NO: 6).

The target gene fragment can be synthesized by a biological company, or the bacteriophage P53 genome can be used as a template for the amplification of the target gene. The disclosure uses the bacteriophage P53 genome as a template to amplify the target gene, and the amplification system is a 50 µL reaction system, which specifically includes the following components: 5 µL of 10× buffer (Mg²⁺ concentration is 20 millimoles per liter abbreviated as mmol/L), 4 µL of deoxynucleotide triphosphates abbreviated as dNTPs (2.5 mmol/L), 2 µL of 53P37-F (10 micromoles per liter abbreviated as µmol/L), 2 µL of 53P37-R (10 µmol/L), 0.5 µL of deoxyribonucleic acid (DNA) template, 0.2 µL of Taq DNA polymerase (5 units per microliter abbreviated as U/µL), and 36.3 µL of double-distilled water (ddH₂O). Polymerase chain reaction (PCR) reaction conditions are as follows: pre-denaturation at 98°C for 5 min, 30 cycles of denaturation at 98°C for 10 seconds (s), annealing at 55°C for 15 s and extension at 72°C for 50 s, and final extension at 72°C for 5 min. After the reaction, PCR amplification products are detected by agarose gel electrophoresis, purified and verified by sequencing, digested with Ncol restriction endonuclease and X*hol* restriction endonuclease, and then connected with the vector pET28a digested with the same N*col* restriction endonuclease and X*hol* restriction endonuclease to obtain the recombinant expression vector pET28a-LysP53. Then, the recombinant expression vector pET28a-LysP53 is transformed into *Escherichia coli* BL21(DE3), and positive clones are selected and verified by sequencing.

### 2.2 Expression and purification of lysin LysP53

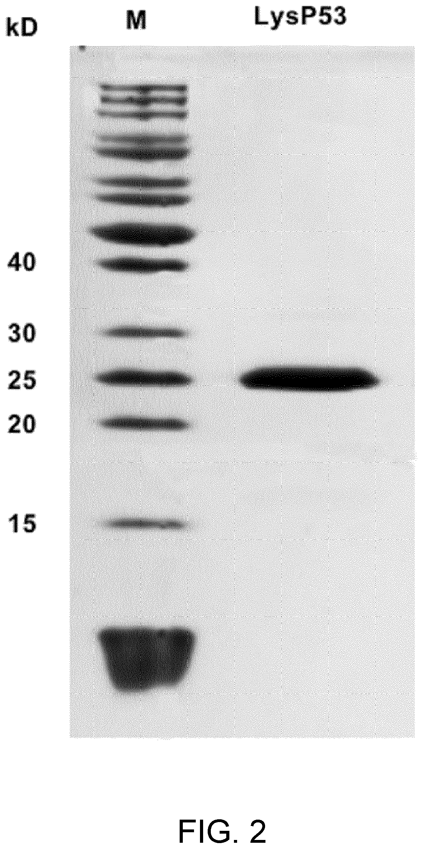

*Escherichia coli* BL21 (DE3) with correct sequencing and containing the recombinant expression vector pET28a-LysP53 is cultured in 500 mL Luria-Bertani (LB) medium containing 50 micrograms per milliliter (µg/mL) kanamycin until the optical density at 600 nm (OD600) is in a range of 0.5-0.6, and then induced with 0.5 millimoles per liter (mM) isopropyl β-D-thiogalactopyranoside (IPTG) (purchased from Thermo Fisher Scientific) for 16 hours (h). Then, the induced product is centrifuged at 4°C and 8000 revolutions per minute (rpm) for 10 min; the cells are collected, rinsed once with 20 mM imidazole, and resuspended in 20 mM imidazole. The resuspended cells are crushed on ice by a cell crusher (also referred to as beads cell disrupter system), centrifuged at 4°C and 8000 rpm for 20 min, the supernatant is filtered by a 0.22 µm filter membrane, then passed through a nickel column for affinity chromatography, and the fragments eluted with 250 mM imidazole are collected. The collected fragments are dialyzed overnight in 20 mM Tris buffer (pH 6.8) at 4°C to obtain the lysin LysP53, and the gel image of sodium dodecyl sulfate-polyacrylamide gel electrophoresis (SDS-PAGE) of the lysin LysP53 after purification is shown in FIG. 2.

As can be seen from FIG. 2, the size of the lysin LysP53 is about 24 kilodaltons (kDa).

### Embodiment 3 Effect of time on activity of the lysin LysP53 lysing A. baumannii WHG40137

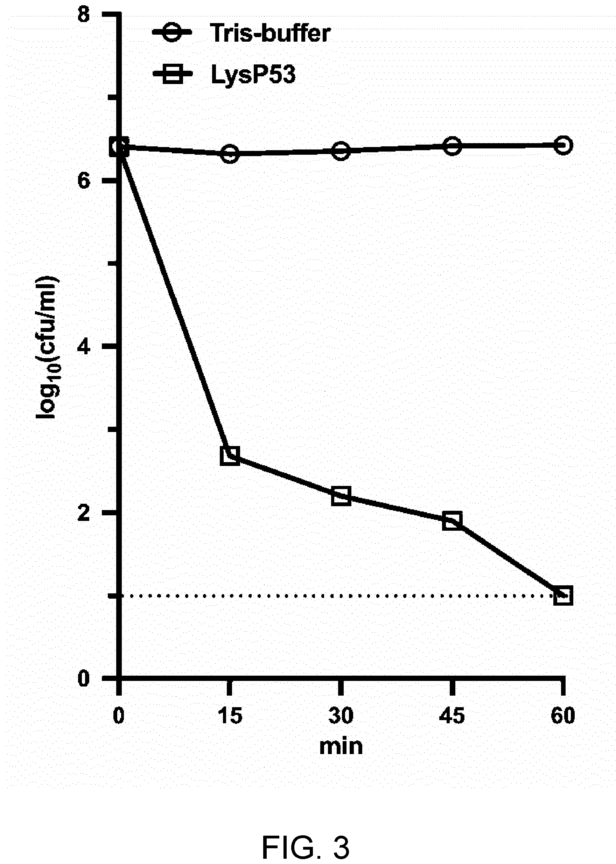

Acinetobacter baumannii (*A. baumannii*) WHG40137 is cultured to logarithmic phase (OD₆₀₀ₙₘ = 0.4-0.6), and the precipitate is collected by low-temperature centrifugation, rinsed once with Tris-hydrochloride (Tris-HCl) buffer, and then the rinsed precipitate is dissolved in the buffer to obtain an *A. baumannii* WHG40137 bacterial solution. The lysin LysP53 prepared in the embodiment 2 is taken and mixed with the *A. baumannii* WHG40137 bacterial solution to make the final concentration of the lysin LysP53 to be 100 micrograms per milliliter (µg/mL), and in this situation, the mixed solution of the same amount of buffer and the above bacterial solution is used as negative control, incubated at 37°C, aliquots were sampled, respectively, at different times and used to count the number of the bacteria in the solutions by plating on agar plates. The results obtained are shown in FIG. 3.

As can be seen from the results in FIG. 3, the concentration of *Acinetobacter baumannii* WHG40137 in the experimental group is greatly reduced after incubation for 15 min, and the concentration of *Acinetobacter baumannii* WHG40137 is reduced to 10 colony-forming units per milliliter (CFU/mL) after incubation for 1 h. The results show that the lysin LysP53 has good lytic activity against *Acinetobacter baumannii* WHG40137.

### Embodiment 4 Results of lytic activity of the lysin LysP53 lysing A. baumannii WHG40137 at different growth periods

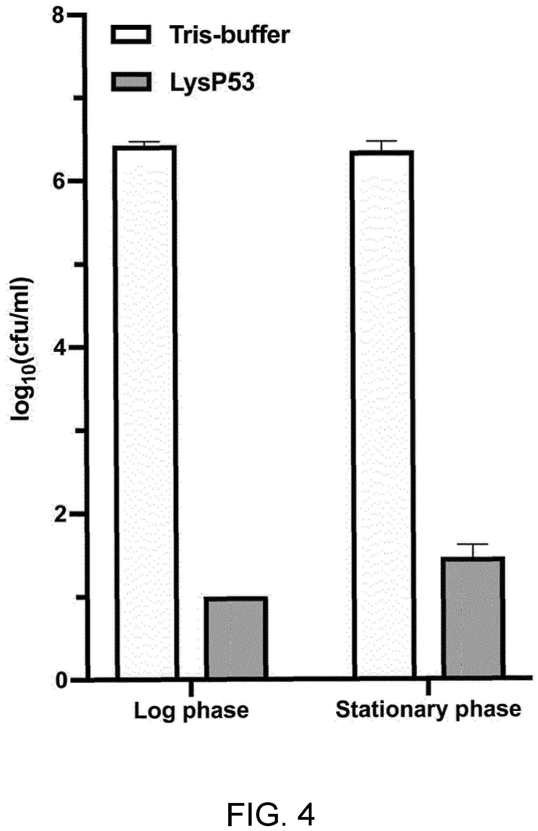

*A. baumannii* WHG40137 is cultured to a logarithmic phase (OD₆₀₀ₙₘ = 0.4-0.6) and a stable phase (OD₆₀₀ₙₘ = 1.2-1.4), and the precipitate is collected by centrifugation at low temperature, rinsed once with Tris-HCl buffer, and then the rinsed precipitate is dissolved in the above buffer to obtain an *A. baumannii* WHG40137 bacterial solution. The lysin LysP53 prepared in the embodiment 2 is taken and mixed with the *A. baumannii* WHG40137 bacterial solution to make the final concentration of the lysin LysP53 to be 100 µg/mL, and in this situation, the mixed solution of the same amount of buffer and the above bacterial solution is used as negative control. After incubation at 37°C for 1 h, the number of the bacteria in the solutions are counted by plating on agar plates. The results are shown in FIG. 4.

It can be seen from the results in FIG. 4 that the lysin LysP53 has higher lytic activity against *A*. *baumannii* WHG40137 in logarithmic phase.

### Embodiment 5 Effect of different pH values on lytic activity of the lysin LysP53 lysing A. baumannii WHG40137

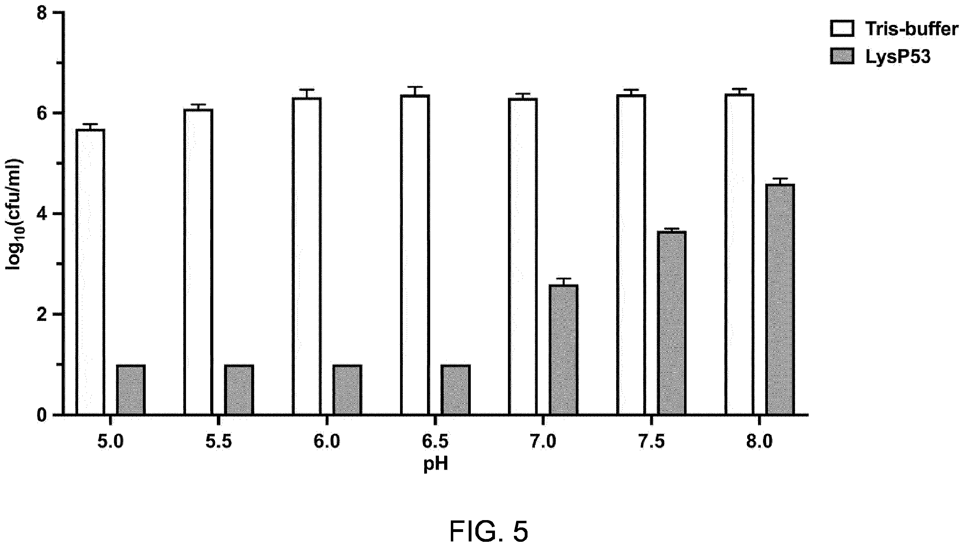

*A. baumannii* WHG40137 is cultured to logarithmic phase (OD₆₀₀ₙₘ = 0.4-0.6), and the precipitate is collected by low-temperature centrifugation, washed once with Tris-HCl buffer, and then resuspended with the above buffers with different pH values (pH 5-8) to obtain *A. baumannii* WHG40137 bacterial solutions. The lysin LysP53 prepared in the embodiment 2 is taken and mixed with the *A. baumannii* WHG40137 bacterial solutions to make the final concentration of the lysin LysP53 to be 100 µg/mL, and in this situation, the mixed solution of the same amount of buffer and the above bacterial solution is used as negative control. After incubation at 37°C for 1 h, aliquots were sampled, respectively, at different times and used to count the number of the bacteria in the solutions by plating on agar plates. The results obtained are shown in FIG. 5.

As can be seen from the results in FIG. 5, when the pH is in a range of 5.0-6.5, the lysin LysP53 has better lytic activity against *A. baumannii* WHG40137, and the concentration of bacterial solution is reduced to 10 CFU/mL. With the increase of pH (7.0-8.0), the lytic activity of the lysin LysP53 lysing *A. baumannii* WHG40137 is decreased. The results show that the lysin LysP53 has higher activity on *Acinetobacter baumannii* WHG40137 under acidic conditions.

### Embodiment 6 Broad spectrum results of the lysin LysP53 lysing different Gram-negative bacterial strains in vitro

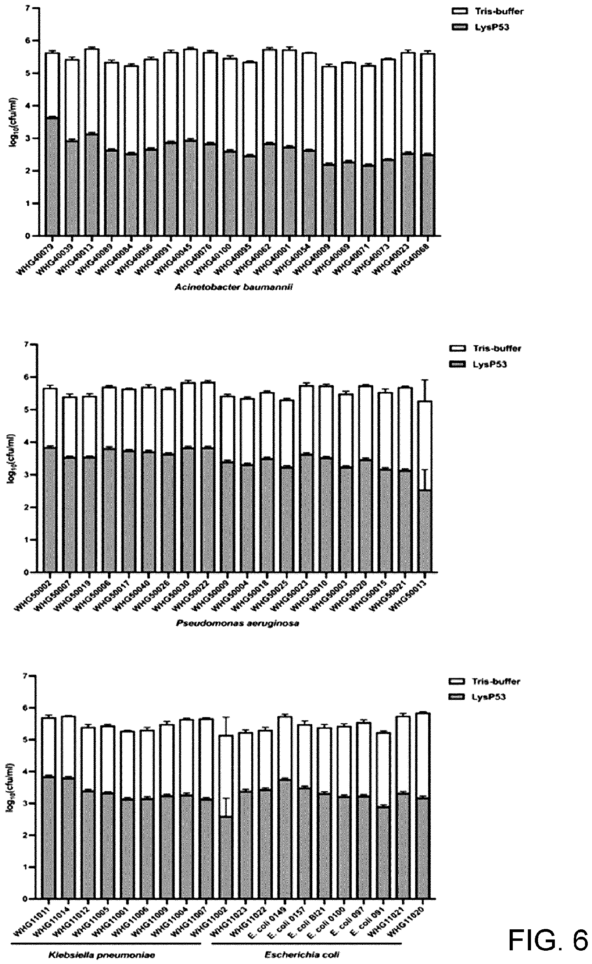

A variety of strains including *Acinetobacter baumannii, Pseudomonas aeruginosa, Klebsiella pneumoniae* and *Escherichia coli* are cultured to a stable phase (OD₆₀₀ₙₘ = 1.2-1.4), and each precipitate is collected by centrifugation at low temperature, rinsed once with Tris-HCl buffer, and then the rinsed precipitate is dissolved in the buffer to obtain a bacterial solution. The lysin LysP53 prepared in the embodiment 2 is taken and mixed with the above bacterial solution to make the final concentration of the lysin LysP53 to be 100 µg/mL, and in this situation, the mixed solution of the same amount of buffer and the above bacterial solution is used as negative control. After incubation at 37°C for 1 h, the number of the bacteria in the solutions are counted by plating on agar plates. The results are shown in FIG. 6.

As can be seen from the results in FIG. 6, the lysin LysP53 has a good lytic effect on a variety of *Acinetobacter baumannii, Pseudomonas aeruginosa, Klebsiella pneumoniae* and *Escherichia coli.* The results show that the lysin LysP53 has a broad spectrum in lysing Gram-negative bacteria.

### Embodiment 7 Effect of different ethylenediaminetetraacetic acid (EDTA) concentrations on the lytic activity of the lysin LysP53 lysing A. baumannii WHG40137

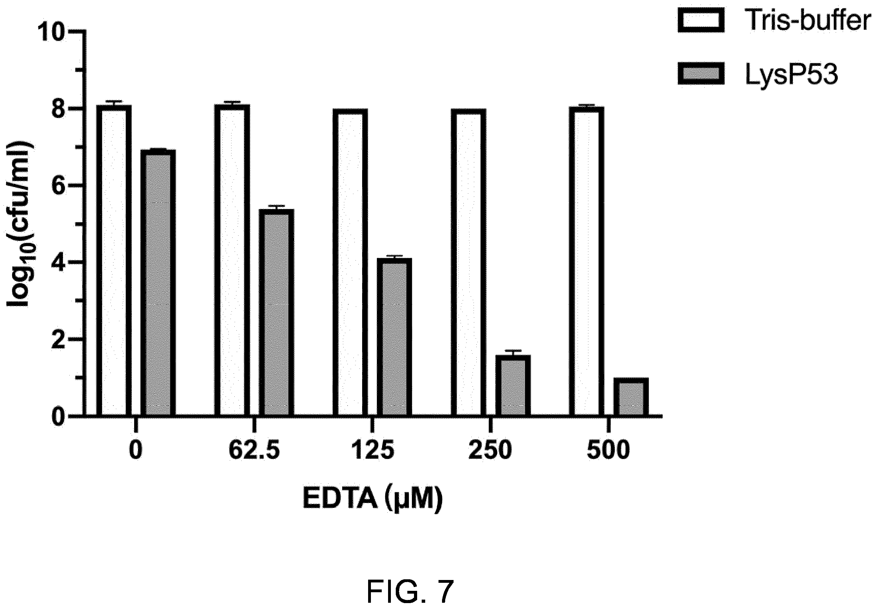

*A. baumannii* WHG40137 is cultured to a stable phase (OD₆₀₀ₙₘ = 1.2-1.4), and the precipitate is collected by centrifugation at low temperature, rinsed once with a Tris-HCl buffer, and then the rinsed precipitate is dissolved in the buffer to obtain an *A. baumannii* WHG40137 bacterial solution. The lysin LysP53 prepared in the embodiment 2 is taken and mixed with the *A. baumannii* WHG40137 bacterial solution to make the final concentration of the lysin LysP53 to be 100 µg/mL, and added with EDTA to the final concentration of 0 µM, 62.5 µM, 125 µM, 250 µM, and 500 µM. In this situation, the mixed solution of the same amount of buffer solution and the above bacterial solution is used as negative control. After incubation at 37°C, aliquots were sampled, respectively, at different times and used to count the number of the bacteria in the solutions by plating on agar plates. The results are shown in FIG. 7.

As can be seen from the results in FIG. 7, with the increase of EDTA concentration, the lytic activity of the lysin LysP53 lysing *A. baumannii* WHG40137 is higher, and the results show that EDTA can significantly promote the lysis of *A*. *baumannii* WHG40137 by the lysin LysP53.

### Embodiment 8 Lytic activity results of the lysin LysP53 lysing A. baumannii WHG40137 on skin surfaces of mice

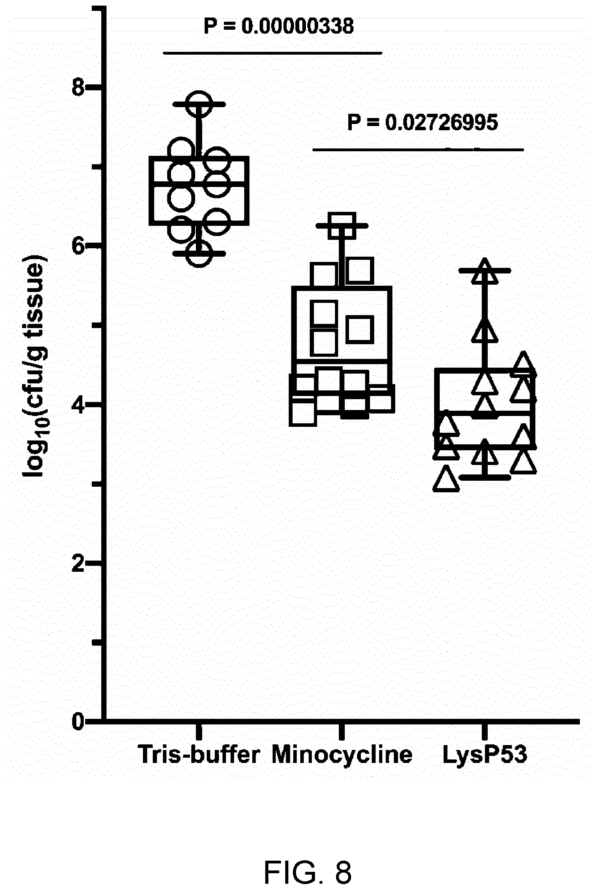

Female BALB/c mice aged 6-8 weeks are anesthetized by intraperitoneal injection of 40 milligrams per kilogram (mg/kg) pentobarbital (also referred to as pentobarbitone), and then shaved with an electric razor to remove 2 square centimeters (cm²) of hair on the back. Partial burn can be achieved by exposing the naked skin to water at 65°C for 12 s. 10 µL of *A. baumannii* WHG40137 with a concentration of 10⁸ CFU/mL is taken and inoculated at the burn site, and after colonization for 24 h, the mice are randomly divided into three groups. In the first group, there are 12 mice, and 14 µg of the lysin LysP53 is added to the burn site of each mouse. In the second group, there are 12 mice, and 4 grams (g) minocycline are added to the burn site of each mouse. In the third group, there are 9 mice, and the same amount of buffer solution is added to the burn site of each mouse. Four hours after treatment, the mice are sacrificed by cervical dislocation and skinned. The infected skin is excised, infiltrated with 1 mL of phosphate buffered saline with Tween^{®} 20 (PBST) containing 0.1% Triton X-100 for 2 min, and homogenized with a tissue cell disruptor (NewZongKe, Wuhan, China), then diluted and coated on an LB plate containing 4 µg/mL of gentamicin and 2 µg/mL of meropenem, and the number of the colonies are counted after overnight culture. The results are shown in FIG. 8.

As can be seen from the results in FIG. 8, compared with minocycline, the lysin LysP53 has a higher lytic activity against *A. baumannii* WHG40137, and there is a significant difference between them.

### Embodiment 9 Results of broad-spectrum of the antimicrobial peptide P104 and the lysin LysP53 lysing different Gram-negative bacterial strains in vitro

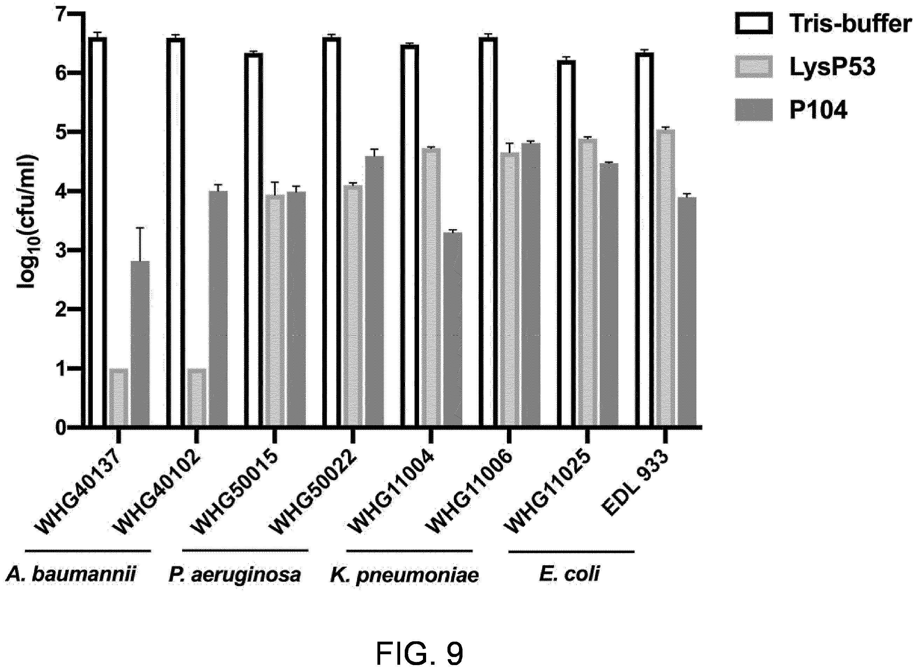

A variety of strains including *Acinetobacter baumannii, Pseudomonas aeruginosa, Klebsiella pneumoniae* and *Escherichia coli* are cultured to a stable phase (OD₆₀₀ₙₘ = 1.2-1.4), and each precipitate is collected by centrifugation at low temperature, rinsed once with Tris-HCl buffer, and then the rinsed precipitate is dissolved in the buffer to obtain a bacterial solution. The lysin LysP53 prepared in the embodiment 2 and the antibacterial peptide P104 prepared in the embodiment 1 are taken and mixed with the above bacterial solution to make the final concentrations of the lysin LysP53 and the antibacterial peptide P104 to be 100 µg/mL respectively. In this situation, the mixed solution of the same amount of buffer and the above bacterial solution is used as negative control. After incubation at 37°C for 1 h, the number of the bacteria in the solutions are counted by plating on agar plates. The results are shown in FIG. 9.

From the results in FIG. 9, it can be seen that the lysin LysP53 and the antibacterial peptide P104 have stronger lytic activity against *Acinetobacter baumannii,* and have certain lytic effects on *Pseudomonas aeruginosa, Klebsiella pneumoniae* and *Escherichia coli.* The results show that the lysin LysP53 and the antibacterial peptide P104 have broad-spectrum lytic activity against Gram-negative bacteria.

The above embodiments are only specific embodiments of the disclosure, which are used to illustrate the technical solutions of the disclosure, but are not limited thereto, and the scope of protection of the disclosure is not limited thereto. Although the disclosure has been described in detail with reference to the above embodiments, it should be understood by those skilled in the art that those skilled in the art may still modify or easily conceive changes to the technical solutions described in the above embodiments, or make equivalent substitutions to some technical features thereof within the technical scope disclosed by the disclosure. However, these modifications, changes or substitutions do not make the essence of the corresponding technical solutions deviate from the spirit and scope of the technical solutions of the embodiment of the disclosure, and should be included in the protection scope of the disclosure.

## Claims

1. An antibacterial peptide P104 with broad-spectrum lytic activity, wherein the amino acid sequence of the antibacterial peptide P104 is shown as SEQ ID NO: 1.

2. The antimicrobial peptide P104 with broad-spectrum lytic activity as claimed in claim 1, wherein the gene sequence of the antimicrobial peptide P104 is shown as SEQ ID NO: 3.

3. A lysin LysP53 with broad-spectrum lytic activity, comprising:
the antibacterial peptide P104 as claimed in claim 1, wherein the amino acid sequence of the lysin LysP53 is shown as SEQ ID NO: 2.

4. The lysin LysP53 with broad-spectrum lytic activity as claimed in claim 3, wherein the gene sequence of the lysin LysP53 is shown as SEQ ID NO: 4.

5. The lysin LysP53 with broad-spectrum lytic activity as claimed in claim 3 or 4, wherein primer sequences configured to amplify the nucleotide sequence of the lysin LysP53 are shown as SEQ ID NO: 5 and SEQ ID NO: 6.

6. A recombinant expression vector, comprising:
the gene sequence of the lysin LysP53 as claimed in claim 4, wherein the recombinant expression vector is a prokaryotic expression vector pET28a-LysP53.

7. A host cell, comprising:
the recombinant expression vector as claimed in claim 6, wherein the host cell is *Escherichia coli* BL21(DE3).

8. A preparation method of the lysin LysP53 with broad-spectrum lytic activity as claimed in claim 3 or 4, comprises:
S1, amplifying a lysin LysP53 gene from a bacteriophage p53 genome;
S2, constructing a recombinant expression vector pet28a-LysP53 expressing the lysin Lysp53 gene;
S3, transforming the recombinant expression vector pET28a-LysP53 into competent cells of *Escherichia coli* BL21(DE3) to obtain a transformed product, and screening the transformed product to obtain engineering bacteria expressing the lysin LysP53 gene;
S4, inducing expression of the engineering bacteria by using isopropyl β-D-thiogalactopyranoside (IPTG) to obtain a recombinant gene expression product; and
S5, purifying and separating the recombinant gene expression product by a nickel column to obtain the lysin LysP53.

9. An application method of the antimicrobial peptide P104 with broad-spectrum lytic activity as claimed in claim 1 or the lysin LysP53 with broad-spectrum lytic activity as claimed in claim 3, comprising:
lysing Gram-negative bacteria by using the antimicrobial peptide P104 with broad-spectrum lytic activity or the lysin LysP53 with broad-spectrum lytic activity.

10. The application method as claimed in claim 9, wherein the Gram-negative bacteria comprises: *Acinetobacter baumannii, Pseudomonas aeruginosa, Klebsiella pneumoniae* and *Escherichia coli.*
